# EUROPEAN PATENT APPLICATION

(11) **EP 4 066 897 A1**
(43) Date of publication of application: **05.10.2022**
(21) Application number: 20893176.6
(22) Date of filing: 20.11.2020
(51) Int. Cl.: A61P 21/00, A61P 43/00, A23K 20/111, A23L 33/10, A61K 31/222

(54) **COMPOSITION FOR PREVENTING DECREASE IN MUSCLE MASS, PREVENTING WEAKNESS OF MUSCULAR POWER, INCREASING MUSCLE MASS OR STRENGTHENING MUSCULAR POWER**

(30) Priority: 27.11.2019 JP 2019214282
(71) Applicant: Suntory Holdings Limited, Osaka 530-8203 (JP)
(72) Inventor: SUZUKI, Toshihide, Soraku-gun, Kyoto 619-0284 (JP); SUDO, Saiko, Soraku-gun, Kyoto 619-0284 (JP); TOMURA, Tomohiko, Soraku-gun, Kyoto 619-0284 (JP)
(74) Representative: Vossius & Partner Patentanwälte Rechtsanwälte mbB
(86) International application number: PCT/JP2020/043305
(87) International publication number: WO 2021/106760

(57) **Abstract**

The present invention aims to provide a composition for reducing muscle mass decline, reducing muscle strength decline, increasing muscle mass, or increasing muscle strength, and a method of reducing muscle mass decline, reducing muscle strength decline, increasing muscle mass, or increasing muscle strength. The present invention relates to, for example, a composition for reducing muscle mass decline, reducing muscle strength decline, increasing muscle mass, or increasing muscle strength, the composition containing 1'-acetoxychavicol acetate.

## Description

### TECHNICAL FIELD

The present invention relates to a composition for reducing muscle mass decline, reducing muscle strength decline, increasing muscle mass, or increasing muscle strength. The present invention also relates to, for example, a method of reducing muscle mass decline, reducing muscle strength decline, increasing muscle mass, or increasing muscle strength.

### BACKGROUND ART

In a country like Japan where the elderly ratio in the population is high, prolongation of healthy life expectancy and improvement of quality of life (QOL) have been agendas. One of the factors that reduce the healthy life expectancy is muscle mass decline with aging or muscle strength decline with aging. Muscle mass decline and muscle strength decline cause an increase in the risk of falling, injuries such as broken bones, and long-term bed rest, for example. In the case of the elderly, a low activity level due to injuries, diseases, or the like leads to a vicious circle with further muscle mass decline and further muscle strength decline, causing locomotive syndrome or the like. Lack of exercise and long rest required after surgery or required for recuperation from a disease, for example, also cause muscle mass decline.

Promotion of protein synthesis is effective in reducing muscle mass decline. The mechanistic target of rapamycin (mTOR) pathway has been reported as being important in protein synthesis. Protein synthesis is promoted by phosphorylation of the threonine (T) 389 residue of ribosomal protein S6 kinase (S6K) and/or the threonine (T) 37/46 residue of eukaryotic translation initiation factor 4E-binding protein 1 (4E-BP1), which are downstream factors of mTOR signal.

A metabolite of leucine, β-hydroxy-β-methylbutyrate (HMB), has been reported as a substance that promotes phosphorylation in the mTOR pathway (Non-Patent Literature 1). It has been reported that resistance training with ingestion of HMB increased muscle mass, as compared to the training without ingestion of HMB (Non-Patent Literature 2).

### CITATION LIST

### - Non-Patent Literature

Non-Patent Literature 1: Kimura et al., NUTRITION RESEARCH 34 (2014), 368-374
Non-Patent Literature 2: Wilson et al., European Journal of Applied Physiology (2014), 114:1217-1227

### SUMMARY OF INVENTION

### - Technical Problem

The present invention aims to provide a composition for reducing muscle mass decline, reducing muscle strength decline, increasing muscle mass, or increasing muscle strength. The present invention also aims to provide a method of reducing muscle mass decline, reducing muscle strength decline, increasing muscle mass, or increasing muscle strength.

### - Solution to Problem

In view of the above issues, the present inventors conducted studies on components that activate the mTOR pathway, and found 1'-acetoxychavicol acetate. 1'-Acetoxychavicol acetate has an action to promote phosphorylation of ribosomal protein S6 kinase (S6K) and eukaryotic translation initiation factor 4E-binding protein 1 (4E-BP1), and is useful in increasing muscle mass, for example.

In other words, the present invention relates to, for example, the following composition for reducing muscle mass decline, reducing muscle strength decline, increasing muscle mass, or increasing muscle strength, but the present invention is not limited thereto.
(1) A composition for reducing muscle mass decline, reducing muscle strength decline, increasing muscle mass, or increasing muscle strength, the composition containing 1'-acetoxychavicol acetate.
(2) The composition according to (1) above, wherein the composition promotes protein synthesis in muscle.
(3) The composition according to (2) above, wherein the composition promotes protein synthesis by activating mTOR pathway.
(4) The composition according to any one of (1) to (3) above, wherein the composition is an oral composition.
(5) The composition according to any one of (1) to (4) above, wherein the composition is a food or beverage.
(6) The composition according to any one of (1) to (5) above, wherein the composition is labeled with one or more function claims selected from the group consisting of "reducing muscle decline", "maintaining muscle", "increasing muscle", "improving muscle", "reducing muscle strength decline", "reducing muscle atrophy", "maintaining muscle strength", "increasing muscle strength", "improving muscle strength", "promoting skeletal muscle hypertrophy", "contributing to muscle synthesis, "maintaining muscle that weakens with aging", and "maintaining muscle strength that weakens with aging".
(7) A method of reducing muscle mass decline, reducing muscle strength decline, increasing muscle mass, or increasing muscle strength, the method including administering 1'-acetoxychavicol acetate.
(8) Use of 1'-acetoxychavicol acetate for reducing muscle mass decline, reducing muscle strength decline, increasing muscle mass, or increasing muscle strength.

### - Advantageous Effects of Invention

The present invention provides, for example, a composition for reducing muscle mass decline, reducing muscle strength decline, increasing muscle mass, or increasing muscle strength. Ingestion of the composition of the present invention can promote protein synthesis, resulting in an effect of reducing muscle mass decline, reducing muscle strength decline, increasing muscle mass, or increasing muscle strength. The present invention provides a method of reducing muscle mass decline, reducing muscle strength decline, increasing muscle mass, or increasing muscle strength.

### BRIEF DESCRIPTION OF DRAWINGS

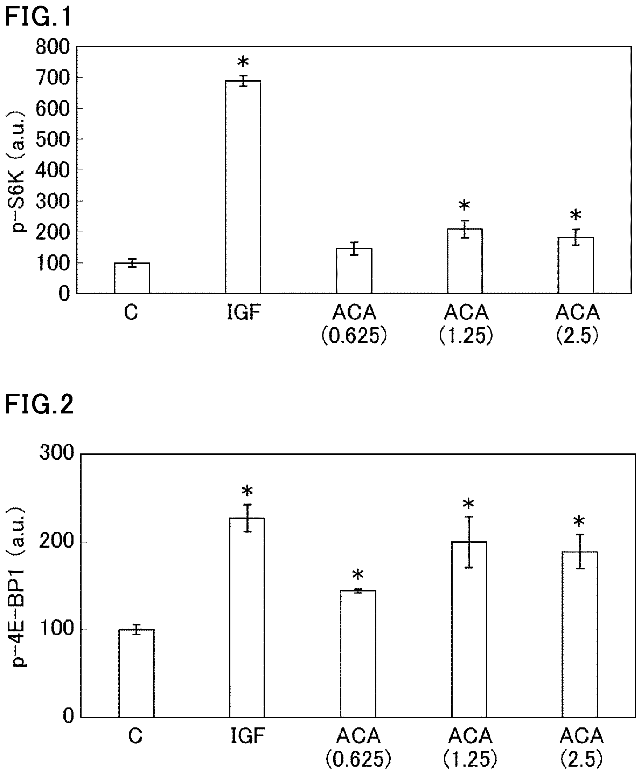

Fig. 1 is a graph showing the phosphorylation level of the T389 residue of S6K of cells, per protein concentration, to which 1'-acetoxychavicol acetate was added. The vertical axis of the graph in Fig. 1 shows relative values of the phosphorylation level, taking the phosphorylation level of the T389 residue of S6K in a negative control (C) as 100.
Fig. 2 is a graph showing the phosphorylation level of the T37/46 residue of 4E-BP1 of cells, per protein concentration, to which 1'-acetoxychavicol acetate was added. The vertical axis of the graph in Fig. 2 shows relative values of the phosphorylation level, taking the phosphorylation level of the T37/46 residue of 4E-BP1 in the negative control (C) as 100.

### DESCRIPTION OF EMBODIMENTS

The composition for reducing muscle mass decline, reducing muscle strength decline, increasing muscle mass, or increasing muscle strength of the present invention contains 1'-acetoxychavicol acetate. The composition for reducing muscle mass decline, reducing muscle strength decline, increasing muscle mass, or increasing muscle strength of the present invention contains 1'-acetoxychavicol acetate as an active ingredient.

Hereinafter, the composition for reducing muscle mass decline, reducing muscle strength decline, increasing muscle mass, or increasing muscle strength of the present invention is also referred to as "the composition of the present invention". In the present invention, the muscle is preferably skeletal muscle.

1'-Acetoxychavicol acetate may be in the D form, L form, or in the form of a mixture of these forms. In the present invention, 1'-acetoxychavicol acetate may be obtained from any source and processed by any method. Naturally occurring or synthetic 1'-acetoxychavicol acetate may be used. Isolated 1'-acetoxychavicol acetate may also be used.

1'-Acetoxychavicol acetate used in the present invention is present in natural products and food and beverages. It is a compound that has a history of consumption. Thus, daily ingestion of 1'-acetoxychavicol acetate, for example, is less likely to cause problems in terms of safety. Thus, the present invention can provide a composition for reducing muscle mass decline, reducing muscle strength decline, increasing muscle mass, or increasing muscle strength, the composition containing a highly safe component.

Promotion of protein synthesis is effective in reducing muscle mass decline, reducing muscle strength decline, increasing muscle mass, or increasing muscle strength.

Protein synthesis is controlled by the mTOR pathway. Activation of the mTOR pathway (e.g., phosphorylation of mTOR pathway signaling factors) promotes protein synthesis. For example, promotion of phosphorylation of S6K and/or 4E-BP1 (downstream factors of mTOR) promotes protein synthesis. Thus, promotion of phosphorylation of S6K and/or 4E-BP1 promotes protein synthesis in muscle, which reduces muscle mass decline or increases muscle mass. A reduction in muscle mass decline or an increase in muscle mass results in an effect of reducing muscle strength decline or increasing muscle strength.

As shown in Examples (described later), 1'-acetoxychavicol acetate promoted phosphorylation of the T389 residue of S6K and the T37/46 residue of 4E-BP1 in C2C12 muscle cells. Thus, 1'-acetoxychavicol acetate has an action to activate the mTOR pathway and has an action to promote protein synthesis in muscle. 1'-Acetoxychavicol acetate has an action to reduce muscle mass decline, an action to reduce muscle strength decline, an action to increase muscle mass, or an action to increase muscle strength, and can be used as an active ingredient for reducing muscle mass decline, reducing muscle strength decline, increasing muscle mass, or increasing muscle strength. 1'-Acetoxychavicol acetate can also be used to promote phosphorylation of S6K and/or 4E-BP1 and to promote protein synthesis.

The composition of the present invention, which contains 1'-acetoxychavicol acetate, can promote protein synthesis in muscle. The composition of the present invention can promote protein synthesis by activating the mTOR pathway, and can be used for such a purpose. In one embodiment, the composition of the present invention can be used to reduce muscle mass decline, reduce muscle strength decline, increase muscle mass, or increase muscle strength by promoting protein synthesis in muscle.

The composition of the present invention produces an effect of reducing muscle mass decline, an effect of reducing muscle strength decline, an effect of increasing muscle mass, or an effect of increasing muscle strength. These effects make it possible, for example, to prevent muscle mass decline, maintain muscle mass, prevent muscle strength decline, maintain muscle strength, improve a condition with reduced muscle mass or reduced muscle strength, or the like. Thus, the composition of the present invention can be used, for example, to prevent muscle mass decline, prevent muscle strength decline, improve a condition with reduced muscle mass, improve a condition with reduced muscle strength, maintain muscle mass, maintain muscle strength, or the like. In one embodiment, the composition of the present invention is suitably used to reduce muscle mass decline or reduce muscle strength decline. The composition of the present invention can be suitably used, for example, to reduce muscle mass decline with aging (e.g., muscle mass decline due to muscle atrophy or the like), reduce muscle strength decline with aging, reduce muscle mass decline due to lack of exercise, reduce muscle strength decline due to lack of exercise, or the like. The muscle mass decline with aging and the muscle strength decline with aging may be muscle mass decline with aging and muscle strength decline with aging among middle-aged people, respectively. The term "middle-aged people" encompasses the elderly. More preferably, the middle-aged people are the elderly. In the present invention, the middle-aged people may be people who are 40 years old and older, for example. The elderly may be people who are 60 years old and older or 65 years old and older, for example.

The muscle mass of animals including humans can be measured by, for example, microPET/CT (positron emission tomography/computed tomography, INVEON, Siemens, USA).

The composition of the present invention can be used to treat a condition or disease likely to be prevented or ameliorated by reducing muscle mass decline, reducing muscle strength decline, increasing muscle mass, or increasing muscle strength. Examples of such a condition or disease include conditions or diseases with reduced muscle mass or reduced muscle strength, such as locomotive syndrome, sarcopenia, frailty, and cachexia (a condition with pathophysiology of severe skeletal muscle atrophy and organ dysfunction due to anorexia and impaired metabolic regulation, caused by debilitating diseases such as cancer and chronic diseases). Frailty refers to physical frailty including muscle mass decline and/or muscle strength decline. In one embodiment, the composition of the present invention can be used to prevent or ameliorate such a condition or disease. In one embodiment, the composition of the present invention may be used by a subject with such a condition or disease.

Herein, the expression "preventing a condition or disease" encompasses preventing the onset of a condition or disease, delaying the onset of a condition or disease, reducing the incidence of a condition or disease, reducing the onset risk of a condition or disease, and the like. The expression "ameliorating a condition or disease" encompasses helping a subject recover from a condition or disease, alleviating a symptom of a condition or disease, delaying or preventing the progress of a condition or disease, and the like. The term "recover" encompasses at least partial recovery.

The composition of the present invention is applicable for both therapeutic use (medical use) and non-therapeutic use (non-medical use). The "non-therapeutic" is a concept that does not include medical activities, i.e., a concept that does not include methods of surgery, therapy, or diagnosis of humans.

The composition of the present invention can be provided in any form such as a food or beverage, a pharmaceutical product, a quasi-pharmaceutical product, feed, or the like. The composition of the present invention may be a food or beverage, a pharmaceutical product, a quasi-pharmaceutical product, feed, or the like by itself, or may be a preparation or material such as an additive used in these products. The composition of the present invention can be provided as an agent or the like, for example, but it is not limited thereto. The agent can be directly provided as a composition, or can be provided as a composition containing the agent. The composition of the present invention can also be referred to as an agent for reducing muscle mass decline, reducing muscle strength decline, increasing muscle mass, or increasing muscle strength.

In one embodiment, the composition of the present invention is preferably an oral composition. The present invention can provide an oral composition having an excellent action to reduce muscle mass decline, reduce muscle strength decline, increase muscle mass, or increase muscle strength. The oral composition may be a food or beverage, an oral pharmaceutical product, or an oral quasi-pharmaceutical product, preferably a food or beverage.

The composition of the present invention may contain an optional additive and an optional component in addition to 1'-acetoxychavicol acetate, as long as the effect of the present invention is not impaired. Such an additive and such a component can be selected according to the form of the composition or the like. Those that can be used generally in food and beverages, pharmaceutical products, quasi-pharmaceutical products, feed, and the like can be used.

When the composition of the present invention is provided as a food or beverage, a component usable in food or beverages (e.g., a food material or an optional food additive) can be added to 1'-acetoxychavicol acetate to provide various types of food and beverages. Non-limiting examples of the food or beverage include general foods or beverages, health foods, health drinks, foods with function claims, foods for specified health uses, dietary supplements, and food and drinks for the sick. The health foods, foods with function claims, foods for specified health uses, dietary supplements, and the like can be used in various dosage forms including fine granules, tablets, granules, powders, capsules, chewable tablets, dry syrups, syrups, liquid formulations, beverages, liquid foods, and the like.

When the composition of the present invention is provided as a pharmaceutical product or a quasi-pharmaceutical product, a pharmacologically acceptable excipient and the like can be added to 1'-acetoxychavicol acetate to provide various dosage forms of pharmaceutical products or quasi-pharmaceutical products. In order to more sufficiently obtain the effect of the present invention, a preferred form of administration of the pharmaceutical product or the quasi-pharmaceutical product is oral administration. The dosage form may be one suitable for administration. Examples of dosage forms for oral administration include solid oral formulations such as tablets, coated tablets, fine granules, granules, powders, pills, capsules, dry syrups, and chewable tablets; and liquid oral formulations such as internal liquid formulations and syrups. Examples of dosage forms for parenteral administration include injection, infusion, ointments, lotions, adhesive skin patches, suppositories, nasal agents, and transpulmonary agents (inhalants). The pharmaceutical product may be for non-human animals.

When the composition of the present invention is provided as feed, a component usable in feed can be added to 1'-acetoxychavicol acetate to provide the feed. Examples of the feed include livestock feed for animals such as cows, pigs, chickens, sheep, and horses; feed for small animals such as rabbits, guinea pigs, rats, and mice; and pet food for animals such as dogs, cats, and birds.

When the composition of the present invention is provided as a food or beverage, a pharmaceutical product, a quasi-pharmaceutical product, feed, or the like, its production method is not limited. Such a product can be produced by a usual method, using 1'-acetoxychavicol acetate.

The amount of 1'-acetoxychavicol acetate in the composition of the present invention can be appropriately set according to the form of the composition or the like. For example, the amount of 1'-acetoxychavicol acetate may be 0.00002 to 20 wt% in the composition. In one embodiment, when the composition of the present invention is provided as an oral composition such as a food or beverage, a pharmaceutical product, or a quasi-pharmaceutical product, the amount of 1'-acetoxychavicol acetate in the composition may be 0.00002 wt% or more, preferably 0.0002 wt% or more, more preferably 0.002 wt% or more, and may be 20 wt% or less, preferably 10 wt% or less, more preferably 2 wt% or less. In one embodiment, the amount of 1'-acetoxychavicol acetate in the composition of the present invention is preferably 0.0002 to 10 wt%, more preferably 0.002 to 2 wt%. The amount of 1'-acetoxychavicol acetate can be measured according to a known method, such as HPLC.

The composition of the present invention can be ingested or administered by a method appropriate to the form. The composition of the present invention is preferably orally administered or orally ingested.

The intake (dosage) of the composition of the present invention is not limited, as long as it is an amount (effective amount) that produces the effect of reducing muscle mass decline, the effect of reducing muscle strength decline, the effect of increasing muscle mass, or the effect of increasing muscle strength. The intake may be appropriately set according to the form of administration, administration method, and the like. For example, when a human (adult) is subjected to oral administration or ingestion, the daily intake of 1'-acetoxychavicol acetate per 60 kg of body weight is preferably 1 mg or more, more preferably 10 mg or more, and preferably 1000 mg or less, more preferably 200 mg or less. In one embodiment, when a human (adult) is subjected to oral administration or ingestion, the daily intake of the composition of the present invention, in terms of 1'-acetoxychavicol acetate, per 60 kg body weight is preferably 1 to 1000 mg, more preferably 10 to 200 mg. Preferably, the above amount of the composition is orally administered or ingested, for example, once a day or in two or three portions a day. When a human (adult) is subjected to ingestion of the composition of the present invention for a purpose of obtaining the effect of reducing muscle mass decline, the effect of reducing muscle strength decline, the effect of increasing muscle mass, or the effect of increasing muscle strength, preferably, the composition of the present invention is orally ingested or administered such that the intake of 1'-acetoxychavicol acetate is in the above ranges.

In one embodiment, preferably, the composition of the present invention contains 1'-acetoxychavicol acetate in an amount, i.e., effective amount, that produces a desired effect of the present invention, in view of the form of administration, administration method, and the like. In one embodiment, for example, when the composition of the present invention is an oral composition such as a food or beverage or an oral pharmaceutical product, the amount of 1'-acetoxychavicol acetate in the daily intake of the composition per adult (60 kg body weight) is preferably 1 to 1000 mg, more preferably 10 to 200 mg.

Continuous ingestion (administration) of 1'-acetoxychavicol acetate is expected to improve the effect of reducing muscle mass decline, reducing muscle strength decline, increasing muscle mass, or increasing muscle strength. In a preferred embodiment, the composition of the present invention is continuously ingested. In one embodiment of the present invention, the composition of the present invention is continuously ingested for preferably four weeks or more, more preferably eight weeks or more, still more preferably 12 weeks or more.

The subject subjected to administration or ingestion of the composition of the present invention (hereinafter, also simply referred to as an "administration subject") is preferably a mammal (a human or non-human mammal), more preferably a human. The administration subject in the present invention is preferably a subject needing or wanting to reduce muscle mass decline, reduce muscle strength decline, increase muscle mass, or increase muscle strength. For example, a preferred subject may be one with reduced muscle mass, one with reduced muscle strength, or one wanting to prevent or ameliorate the above-described condition or disease with reduced muscle mass or reduced muscle strength. In one embodiment, the administration subject of the composition of the present invention is preferably a middle-aged person, more preferably an elderly person. In one embodiment, the composition of the present invention can be used by a healthy subject for purposes such as preventing a condition or disease likely to be prevented or ameliorated by reducing muscle mass decline, reducing muscle strength decline, increasing muscle mass, or increasing muscle strength.

A package, container, package insert, or the like of the composition of the present invention may include one or more descriptions regarding usage, types of active ingredients, the above-described effects, instructions for use (e.g., ingestion method or administration method), and the like. The composition of the present invention may be labeled as having an action to reduce muscle mass decline, an action to reduce muscle strength decline, an action to increase muscle mass, an action to increase muscle strength, or an action based on these actions. For example, the composition of the present invention may be labeled with one or more function claims such as "reducing muscle decline", "maintaining muscle", "increasing muscle", "improving muscle", "reducing muscle strength decline", "reducing muscle atrophy", "maintaining muscle strength", "increasing muscle strength", "improving muscle strength", "promoting skeletal muscle hypertrophy", "contributing to muscle synthesis", "maintaining muscle that weakens with aging", "maintaining muscle strength that weakens with aging", and the like.

In one embodiment of the present invention, preferably, the composition of the present invention is a food or beverage labeled with one or more function claims described above. These labels may be labels indicating use for obtaining these functions. These labels may be attached to the composition itself or a container or a package of the composition.

The present invention also encompasses the following method and use:
a method of reducing muscle mass decline, reducing muscle strength decline, increasing muscle mass, or increasing muscle strength, the method including administering 1'-acetoxychavicol acetate; and
use of 1'-acetoxychavicol acetate for reducing muscle mass decline, reducing muscle strength decline, increasing muscle mass, or increasing muscle strength.

The method may be therapeutic or non-therapeutic. The use may be therapeutic or non-therapeutic.

Administration of 1'-acetoxychavicol acetate to a subject activates the mTOR pathway, which in turn can promote protein synthesis in muscle. In one embodiment, 1'-acetoxychavicol acetate can be used to promote protein synthesis in muscle so as to reduce muscle mass decline, reduce muscle strength decline, increase muscle mass, or increase muscle strength. The use may be use of 1'-acetoxychavicol acetate to reduce muscle mass decline, reduce muscle strength decline, increase muscle mass, or increase muscle strength by promoting protein synthesis in muscle. In one embodiment, 1'-acetoxychavicol acetate can be used to prevent or ameliorate a condition or disease such as locomotive syndrome, sarcopenia, frailty, cachexia, or the like by reducing muscle mass decline, reducing muscle strength decline, increasing muscle mass, or increasing muscle strength.

In the method and the use, preferably, 1'-acetoxychavicol acetate is administered to (ingested by) a subject at least once a day, for example, one to several times (e.g., two to three times) a day. In the method and the use, preferably, 1'-acetoxychavicol acetate is orally administered (ingested). The use is preferably for humans or non-human mammals, more preferably for humans.

The method and the use only require use of 1'-acetoxychavicol acetate in an amount (effective amount) that produces a desired action. A preferred dosage, administration subject, and the like of 1'-acetoxychavicol acetate are as described above for the composition of the present invention. 1'-Acetoxychavicol acetate may be administered as is, or may be administered in the form of a composition containing 1'-acetoxychavicol acetate. For example, the above-described composition of the present invention may be administered.

In one embodiment, the present invention also encompasses use of 1'-acetoxychavicol acetate for producing a composition for reducing muscle mass decline, reducing muscle strength decline, increasing muscle mass, or increasing muscle strength. The composition for reducing muscle mass decline, reducing muscle strength decline, increasing muscle mass, or increasing muscle strength, its preferred embodiments, and the like are as described above for the composition of the present invention and its preferred embodiments. The present invention also encompasses 1'-acetoxychavicol acetate for use in reducing muscle mass decline, reducing muscle strength decline, increasing muscle mass, or increasing muscle strength. The present invention also encompasses 1'-acetoxychavicol acetate for use in preventing or ameliorating locomotive syndrome, sarcopenia, frailty, or cachexia.

### EXAMPLES

The present invention is described in further detail below with reference to examples, but these examples are not intended to limit the scope of the present invention.

### <Example 1>

A mouse myoblast line (C2C12 cells available from ATCC) was used. The C2C12 cells were seeded onto a 96 well plate at 2 × 10⁴ cells/plate, and cultured in high glucose normal DMEM (available from Sigma-Aldrich) containing 10% fetal bovine serum (FBS, available from Sigma-Aldrich) and 1% antibiotics (an antibiotic-antifungal mixture available from Nacalai Tesque Inc.) at 37°C for three days. After the C2C12 cells were grown to confluence, the following experiment was performed.

The FBS-containing DMEM medium was replaced with high glucose normal DMEM (available from SIGMA-Aldrich) free of FBS and containing 1% antibiotics (hereinafter also referred to as an "FBS-free medium"), and the cells were further cultured for 20 hours.

The FBS-free medium was replaced with an FBS-free medium to which D,L-1'-acetoxychavicol acetate (ACA) (available from Abcam) or IGF-1 (recombinant analog human available from Cell Sciences) was added. The final concentration of ACA was 0.625 µg/mL, 1.25 µg/mL, or 2.5 µg/mL. IGF-1 was used as a positive control, and the final concentration of IGF-1 in the medium was 100 ng/mL. Separately from the medium to which ACA or IGF-1 was added, a medium (control medium) was prepared by adding dimethyl sulfoxide (DMSO) to the FBS-free medium to a final concentration of 0.1% (v/v), and the FBS-free medium was replaced with the control medium (negative control).

The C2C12 cells were cultured in the FBS-free medium to which ACA was added, the FBS-free medium to which IGF-1 was added (positive control), or the FBS-free medium to which DMSO was added (negative control) for one hour. Subsequently, a cell lysis buffer (Cell Signaling Technology) was added to recover the cells. The cell lysis buffer was used after phenylmethylsulfonyl fluoride (PMSF) was added thereto to a final concentration of 1 mM.

Using ELISA according to a protocol attached to ELISA Kits (Cell Signaling Technology), the phosphorylation level of the T (Thr) 389 residue of ribosomal protein S6 kinase (S6K) and the phosphorylation level of the T (Thr) 37/46 residue of eukaryotic translation initiation factor 4E-binding protein 1 (4E-BP1) were measured, and the amount of phosphorylated S6K (T389) and the amount of phosphorylated 4E-BP1 (T37/46) per protein concentration in samples were calculated. Dunnet's test was performed for significance testing (significance level: p < 0.05, vs. negative control).

Fig. 1 and Fig. 2 show the results. Fig. 1 is a graph showing the phosphorylation level of the T389 residue of S6K of cells, per protein concentration, to which 1'-acetoxychavicol acetate (ACA) was added. Fig. 2 is a graph showing the phosphorylation level of the T37/46 residue of 4E-BP1 of cells, per protein concentration, to which 1'-acetoxychavicol acetate (ACA) was added. The vertical axis of the graph in each of Fig. 1 and Fig. 2 shows relative values of the phosphorylation level, taking the phosphorylation level in the negative control (C) as 100.

In Fig. 1 and Fig. 2, "C" indicates the negative control, "IGF" indicates IGF-1 in the positive control, and "ACA" indicates 1'-acetoxychavicol acetate (0.625 µg/mL, 1.25 µg/mL, or 2.5 µg/mL). The results in Fig. 1 and Fig. 2 are all represented in means ± standard error with n = 4 (*: p < 0.05, vs. negative control). ACA (0.625), ACA (1.25), and ACA (2.5) indicate an ACA concentration in the medium of 0.625 µg/mL, 1.25 µg/mL, and 2.5 µg/mL, respectively.

As is clear from Fig. 1 and Fig. 2, 1'-acetoxychavicol acetate promoted phosphorylation of S6K and 4E-BP1. These results clearly show that 1'-acetoxychavicol acetate has an action to promote protein synthesis.

## Claims

1. A composition for reducing muscle mass decline, reducing muscle strength decline, increasing muscle mass, or increasing muscle strength, the composition comprising:
1'-acetoxychavicol acetate.

2. The composition according to claim 1,
wherein the composition promotes protein synthesis in muscle.

3. The composition according to claim 2,
wherein the composition promotes protein synthesis by activating mTOR pathway.

4. The composition according to any one of claims 1 to 3,
wherein the composition is an oral composition.

5. The composition according to any one of claims 1 to 4,
wherein the composition is a food or beverage.

6. The composition according to any one of claims 1 to 5,
wherein the composition is labeled with one or more function claims selected from the group consisting of "reducing muscle decline", "maintaining muscle", "increasing muscle", "improving muscle", "reducing muscle strength decline", "reducing muscle atrophy", "maintaining muscle strength", "increasing muscle strength", "improving muscle strength", "promoting skeletal muscle hypertrophy", "contributing to muscle synthesis", "maintaining muscle that weakens with aging", and "maintaining muscle strength that weakens with aging".

7. A method of reducing muscle mass decline, reducing muscle strength decline, increasing muscle mass, or increasing muscle strength, the method comprising:
administering 1'-acetoxychavicol acetate.

8. Use of 1'-acetoxychavicol acetate for reducing muscle mass decline, reducing muscle strength decline, increasing muscle mass, or increasing muscle strength.
